Europäisches Patentamt

European Patent Office

Office européen des brevets

(19)

(11) Publication number: **0 175 468**
A2

(12) **EUROPEAN PATENT APPLICATION**

(21) Application number: **85305660.4**

(22) Date of filing: **09.08.85**

(51) Int. Cl.⁴: **A 61 K 31/23**

(30) Priority: **10.08.84 ZA 845267**

(43) Date of publication of application: **26.03.86**
**Bulletin 86/13**

(84) Designated Contracting States: **AT BE CH DE FR GB IT LI LU NL SE**

(71) Applicant: **SENTRACHEM LIMITED, 20 Anderson Street, Marshalltown Transvaal 2001 (ZA)**

(72) Inventor: **Booyens, Jakobus, Plot 45, Patryshoek (ZA)**

(74) Representative: **Brown, John David et al, FORRESTER & BOEHMERT Widenmayerstrasse 4/I, D-8000 München 22 (DE)**

(54) **Eicosanoids for use in cancer therapy.**

(57) The invention comprises a method of normalising cellular eicosanoid balance by administering to a warm blooded animal an effective amount of a composition chosen from the group comprising eicosapentanoic acid (EPA), docosahexanoic acid (DHA) a mixture of EPA and DHA and a mixture of EPA, DHA and GLA. The invention also relates to compositions for normalising cellular eicosanoid balance for the preventing or treatment of cancer.

EP 0 175 468 A2

## FIELD OF THE INVENTION

This invention relates to substances and compositions containing such substances for use in the treatment of cancerous conditions.

## BACKGROUND

In 1980 Horrobin (The Reversibility of Cancer: The Relevance of Cyclic AMP, Calcium, Essential Fatty Acids and Prostaglandin $E_1$ Med. Hypotheses 1980, Vol. 6, pages 469 to 486) dealt extensively with metabolic abnormalities common to almost all cancer cells, and with possible causative factors for these. Horrobin concludes that a metabolic abnormality in the synthesis of the prostaglandins thromboxande $A_2$ ($TXA_2$) and prostaglandin $E_1$ ($PGE_1$) is the final factor which allows an initiated cancer cell to express its abnormality, that is to divide ad infinitum. Horrobin further proposed (on the basis of evidence present in the general literature) that the defect which leads to the abnormality in the synthesis of $TXA_2$ and $PGE_1$ is an inhibition of the enzyme delta-6-desaturase. This enzyme converts the essential fatty acid linolenic acid (LA), to gamma linolenic acid (GLA) in all normal cells of the body. GLA is further metabolised to dihomo-gamma-linolenic acid (DGLA) which in turn is converted to prostaglandins of the 1-series, which includes $PGE_1$.

Horrobin recognised that $PGE_1$ and $TXA_2$ are potent intracellular regulators of the biochemistry of all normal cells, but that $TXA_2$ however cannot function in the absence of $PGE_1$. Horrobin surmised that a deficiency of $PGE_1$ and thus disabled $TXA_2$ will cause abnormal metabolism of the cell, of sufficient magnitude to trigger off uncontrolled division of potential cancer cells.

Horrobin thus proposed that inter alia a GLA supplement should be given to cancer patients receiving conventional treatment, in order to test his hypothesis, namely, that in bypassing the metabolic block caused by an inhibited delta-6-desaturase (d-6-d) activity, it should be possible to normalise cancer cells by reverse transformation.

In European Patent Application 0 037 Horrobin claims a mixture of GLA and thioproline for the treament of cancer.

OBJECT OF THE INVENTION

It is an object of the present invention to provide compositions for the treatment of cancer by taking into consideration the production of one or more normalised mixtures of eicosanoids.

According to the invention a method of normalising cellular eicosanoid balance by administration of eicosapentanoic acid (EPA) and/or docosahexanoic acid (DHA).

In a preferred form of the invention EPA or DHA or a mixture of EPA and DHA is administered in a number of possible forms such as, for example, capsules , tablets or other convention pharmaceutical forms, or in admixture with foodstuffs, beverages and the like.

It will be appreciated that suitable salts, derivatives, or chemical analogues of the above substances are also in the scope of the present invention. In particular the magnesium and zinc salts are important.

The substance or composition may be provided in unit dosage form, eg for daily or twice-daily administration, such as in tablets or capsules. In each capsule the active ingredient may be solution, as described above, or it may be in the form of a tablet or particulate mixture, comprising the active ingredient together with a solid diluent or carrier. A unit dosage for daily administration typically for a person of 50 to 100 kg body weight, may contain up to 1000 mg of active ingredient.

Suitable solvents for the active ingredients comprise plant oils, isotonic saline solutions or any other lipid or aqueous solvents suitable for human intake.

The invention will now be described and illustrated by way of the following examples which includes both in vitro and in vivo studies:

IN VITRO STUDIES ON CANCER CELLS IN CULTURE

EXAMPLE 1

Human osteogenic sarcoma cells were seeded into 50 ml Greiner plastic flasks and maintained in the normal manner.

The cells were cultured for three weeks in the presence of

i)      growth medium only - control

ii)     growth medium & 10   1 $Na_2CO_3$/ml added every second day

iii)    growth medium & 20   g EPA/ml medium added every second day

iv)     growth medium & 20   g oleic acid/ml added every second day

v)      growth medium &  5   g $PGE_1$/ml added every second day

vi)    growth medium & 5   g $PGA_1$/ml added every second day

vii)   growth medium & 5   g $PGF_{1a}$/ml added every second day.

At the end of three weeks the culture flasks were stained with a 0,1% Amidoschwarz stain solution.

RESULTS

At the end of the three weeks period the initially seeded osteogenic sarcoma cells had established colonies of various sizes almost covering the entire floor of the culture flasks of the control and the $Na_2CO_3$ supplemented flasks.

Oleic acid supplemented cultures achieved much greater growth as control cultures.

$PGE_1$ and $PGA_1$ cultures achieved about 25% of the growth of control cultures.

$PGF_{1 a}$ cultures achieved about the same growth as the control cultures.

The EPA supplemented cultures were completely devoid of any colonies in 500, 1 000 and 2 000 cell density cultures.

$PGE_1$ and $PGA_1$ had a more pronounced growth suppressive effective whilst $PGF_{1a}$ had no effect at all on cancer cell growth. In contrast, EPA had a complete growth suppressive effect.

The results would suggest that uncontrolled cell division in cancer cells could be the result of abnormalities in the concentration of some of the prostaglandins in such cells, resulting from a block in their synthesis from precursor fatty acids. Such abnormalities are evidently eliminated by supplying the cancer cells with EPA, the substrate from which the required prostaglandins can be synthesized in their required concentrations. Once this can be achieved by cancer cells, their uncontrolled division is apparently totally checked.

EXAMPLE 2

In view of the surprising observation reported in example 1, that EPA (the essential fatty acid intermediate prostaglandin precursor of the 3-series prostaglandins, prostacyclin, thromboxane A3 and leukotrienes, was also investigated - EPA being derived from the naturally occuring essential fatty acid $\gamma$-linolenic acid (C 18:3 W3) by the action of d-6-d to give octadecatetraeonic acid (C 18:4 W3), which undergoes chain elongation to eicosatetraeonic acid (C20:4 W3), which in turn gives rise to eicosapentaenoic acid following the action of delta-5-desaturase) supplementation by 40 g/ml medium of mg63 osteogenic sarcoma cells completely suppresses proliferation and colony formation of the cells in culture, this experiment was repeated in order to confirm the observation.

In addition the final product of $\gamma$-linolenic acid metabolism, which is DCAA was also added to osteogenic cells in culture.

PROCEDURE

MG63 Human osteogenic sarcoma cells were seeded in culture flasks as described in example 1.
2 000 cells were seeded in each flask. Duplicate sets of flasks were used for each of the fatty acids tested. The following fatty acids dissolved in standard growth medium were added to the cells in culture, after allowing 2 days for cell attachment, and again after a further 3 days. Each culture therefore had only 2 additions of the relevant fatty acid. The cells were stained and examined at the end of 7 days in culture.

1. Culture medium only - control.
2. 5, 10, 20, 40, 60, 80 and 100 g oleic acid respectively /ml culture medium (Oleic acid (OA) is an 18 C fatty acid with one unsaturated bond in the omega-9 position. It is therefore structurally nearly identical to either LA and $\gamma$-LA with the

singular exception of the number of double bonds contained in the molecule. On account of the latter difference, OA, unlike LA and ɤ-LA cannot give rise to eicosanoids. It is therefore considered to be an excellent fatty acid to use as a control when investigating the effects of the eicosanoids.

3. 5, 10, 20, 40, 60, 80 and 100  g/ml EPA  respectively /ml culture medium.

4. 5, 10, 20, 40, 60, 80 and 100  g/ml DHA respecitvely /ml culture medium.

RESULTS

1. OA supplemented cells achieved greater densities of colonies for all levels of supplementation between 5 and 100  g/ml,    as did the controls with culture medium only.

2. EPA and  DHA exhibited an almost equal, progressive, suppressive action on the proliferation and colony formation of the MG63 osteogenic carcinoma cells.

3. EPA and  DHA completely suppressed cell growth and colony formation at levels of supplementation above 40  g/ml culture medium.

Not a single cell or colony of cells could be found on microscopic  examination of the cultures which had been supplemented with either EPA or DHA at supplementation levels between 10 and 100 g/ml.

It would therefore appear that the fatty acid metabolites, EPA and DHA have the ability to individually arrest and suppress cancer cell growth. It would further appear that any one of these eicosanoid precursor fatty acids separately or in combination could be used for the treatment of cancer.

These results have been confirmed using three other cancer cell types i.e. larynx carcinoma hepatoma    (liver cancer)

melanoma    (skin cancer).

## TABLE 1

The effect of supplementing human larynx carcinoma cells in culture with varying concentrations of oleic acid and eicosapentaenoic acid on the rate of proliferation. Cells were seeded in a concentration of 0,0698 x $10^6$/ml on day 1 of the experiment. Growth media containing the various fatty acid supplements were added to the cultures on days 3 and 5 of the experimental period and cell counts were made on day 8 of the experimental period. Control cultures received standard growth medium only.

| | Supplement concentraation g/ml medium | Mean Cell count x $10^6$ | SD | p-value | Difference between control and supplemented counts |
|---|---|---|---|---|---|
| | Control | 0,2098 | 0,057 | | |
| OA | 20 | 0,36 | 0,163 | 0,05 | ns |
| | 40 | 0,41 | 0,091 | 0,05 | ns |
| | 60 | 0,21 | 0,49 | 0,05 | ns |
| EPA | 20 | 0,26 | 0,42 | 0,05 | ns |
| | 40 | 0,07 | 0,015 | 0,01 | hs |
| | 60 | 0,08 | 0,0357 | 0,01 | hs |

TABLE II

The effect of supplementing human larynx carcinoma cells in culture with combinations in equal proportion of $\gamma$-linolenic acid, and eicosapentanoic acid; prostaglandins E, and A,; prostaglandins F, and $F_2$ ; and with docosahexaenoic acid in varying concentrations on the rate of proliferation. Cells were seeded in a concentration of 0,025 x $10^6$/ml on day 1 of the experiment. The various supplements were added on days 3 and 5 of the experimental period and cell counts were made on day 8.

| Supplement and concentration g/ml medium | Mean Cell count x $10^6$ | SD | p-value | Difference between control and supplemented counts |
|---|---|---|---|---|
| Control | 0,33 | 0,006 | | |
| GLA) 20<br> + ) | 0,22 | 0,005 | 0,01 | hs |
| EPA) 60 | 0,09 | 0,009 | 0,01 | hs |
| $PGE_1$ )<br> + ) 5<br>$PGA_1$ ) | 0,19 | 0,016 | 0,01 | ,hs |
| $PGE_1$ )<br> + ) 5<br>$PGF_2$ ) | 0,33 | 0,009 | 0,05 | ns |
| EPA) 20<br> + | 0,13 | 0,018 | 0,01 | hs |
| D HA )40 | 0,06 | 0,005 | 0,01 | hs |
| D HA )60 | 0,009 | 0,003 | 0,01 | hs |

Results in respect of hepatoma and melanoma were very
similar to the above experiments on larynx carcinoma

In all of the above experiments, duplicate experiments
were conducted using normal MDBK cells in culture.
It is important to note that none of the EFA's
suppressed the growth of the MDBK cells. The growth
suppressive action of the EFA's is therefore very
specific for cancer cells.

A number of human cancer patients who were described as terminal cases following failure of conventional chemotherapeutic and radiation therapeutic procedures responded excellently to daily dietary supplementation (1.6 gm GLA + 1.6g EPA + 0,5g DHA daily). These open trials on terminal patients are being continued.

For example, subject A (age about 55) was suffering from oesophagal cancer (considered a terminal case). He was put on to an EPA/ DHA /GLA supplement as described above, following total removal of his oesophagus and massive metasteses in his thoracic cavity. After six months, A was apparently healthy and back at work.

Subject B (age 35) was suffering from a brain tumour. No tumour removal was recommended and he was expected to live for less than a month.

This was October 1982. His diet was supplemented with GLA/EPA/ DHA . He recovered systematically and is now back at work and drives his own car. The tumour diameter has reduced by 80%, and is still regressing.

Subject C (age 26) had a large primary liver cancer. In April 1983 his diet was supplemented with EPA/ DHA./GLA. The tumour size has and is still regressing substantially and he is back at work. (Primary liver cancer patients have a mean survival time of about 40 days post positive diagnosis),

Subject D (age 60) suffered from unilateral larynx carcinoma and was expected to live for not more than a few months. He is still (after more than a year) receiving a dietary supplement of EPA/DHA/GLA and there has been total regression of the nodule and D is leading a normal life.

In two examples, subjects E and F (ages 55 and 50) suffering from mesothelioma were both given only a short while to live. They are now apparently healthy following six months of dietary supplement.

Further experiments were conducted in relation to the effect of EPA, DHA and mistures thereof, and such mixtures with GLA and were compared with controls and also with GLA on its own. The results of these experiments are given in the following Table.

EFFECT OF DIFFERENT FFA/S COMBINED ON CELL GROWTH     DATE : 13/6/84

CELL LINE : BL-6 # 27

The features disclosed in the foregoing description, in the following claims may, both separately and in any combination thereof, be material for realising the invention in diverse forms thereof.

CLAIMS:

1.

A method of normalising cellular eicosanoid balance including the step of administrating to a warm blooded animal an effective dose of a composition including an effective amount of a substance chosen from the group comprising eicosapentanoic acid (EPA), docosahexanoic acid (DHA) a mixture of EPA and DHA, and a mixture of EPA, DHA and GLA.

2.

The method as claimed in claim 1 in which the composition comprises unit dosage for daily administration of less than 1000 mg of active ingredient or ingredients per 50 to 100 Kg body weight.

3.

The method of claim 1 in which the substances are in the form of their magnesium or zinc salts.

4.

A composition for normalising cellular eicosanoid balance for the prevention or treatment of cancer including a a composition having an effective amount of a substance chosen from the group comprising eicosapentanoic acid (EPA), docosahexanoic acid (DHA) a mixture of EPA and DHA, and a mixture of EPA, DHA and GLA.

5. DHA, or a pharmaceutically acceptable salt thereof or DHA or a pharmaceutically acceptable salt thereof with EPA or a pharmaceutically acceptable salt thereof and/or GLA or a pharmaceutically acceptable salt thereof for use as an active therapeutic substance.

6. DHA, or a pharmaceutically acceptable salt thereof or DHA or a pharmaceutically acceptable salt thereof with EPA or a pharmaceutically acceptable salt thereof and/or GLA or a pharmaceutically acceptable salt thereof for use in the prevention or treatment of cancer.

7. The use of DHA or DHA with EPA and/or GLA in the manufacture of a medicament to prevent or treat cancer.

8. The use of DHA or DHA with EPA and/or GLA in the manufacture of a medicament to prevent or treat cellular eicosanoid imbalance.

9. A use according to claim 7 or 8, wherein a pharmaceutically acceptable salt of one or more of DHA, EPA and GLA is used.